(19) Europäisches Patentamt
European Patent Office
Office européen des brevets

(11) Veröffentlichungsnummer: **0 314 154**
**A2**

(12) **EUROPÄISCHE PATENTANMELDUNG**

(21) Anmeldenummer: 88117961.8

(22) Anmeldetag: 28.10.88

(51) Int. Cl.4: C07D 491/048 , C07D 495/04 , A61K 31/435 , //(C07D491/048,307:00,221:00), (C07D495/04,333:00,221:00)

(30) Priorität: 29.10.87 DE 3736664

(43) Veröffentlichungstag der Anmeldung: 03.05.89 Patentblatt 89/18

(84) Benannte Vertragsstaaten: AT BE CH DE ES FR GB GR IT LI LU NL SE

(71) Anmelder: BOEHRINGER INGELHEIM KG
Postfach 200
D-6507 Ingelheim am Rhein(DE)

(84) BE CH DE ES FR GR IT LI LU NL SE AT

(71) Anmelder: BOEHRINGER INGELHEIM INTERNATIONAL G.M.B.H.
Postfach 20
D-6507 Ingelheim am Rhein(DE)

(84) GB

(72) Erfinder: Harreus, Albrecht, Dr.
Teichgasse 13
D-6700 Ludwigshafen(DE)
Erfinder: Weber, Karl-Heinz, Dr.
Kaiser-Karl-Strasse 11
D-6535 Gau-Algesheim(DE)
Erfinder: Walther, Gerhard, Dr.
Pfarrer-Heberer-Strasse 37
D-6530 Bingen(DE)
Erfinder: Stransky, Werner, Dr. Dipl.-Chem.
Im Hippel 24
D-6535 Gau-Algesheim(DE)
Erfinder: Kuhn, Franz Josef, Dr.
Beethovenstrasse 11
D-6535 Gau-Algesheim(DE)
Erfinder: Schingnitz, Günter, Dr.
Unter den Gärten 18
D-6550 Bad Kreuznach 14(DE)
Erfinder: Ensinger, Helmut, Dr.
Magdeburger Strasse 54
D-6507 Ingelheim(DE)

(54) **Tetrahydro-furo- und -thieno[2,3-c]pyridine, ihre Verwendung als Arzneimittel und Verfahren zu ihrer Herstellung.**

(57) Die Erfindung betrifft neue Tetrahydro-furo- und -thieno[2,3-c]pyridine, Verfahren zu ihrer Herstellung und ihre Verwendung als Arzneimittel mit cholinomimetischen Eigenschaften.

EP 0 314 154 A2

Xerox Copy Centre

# Tetrahydro-furo- und -thieno[2,3-c]pyridine, ihre Verwendung als Arzneimittel und Verfahren zu ihrer Herstellung

Die Erfindung betrifft die Verwendung von Tetrahydro-furo- und -thieno[2,3-c]pyridinen sowie deren Säureadditionssalze als Arzneimittel, insbesondere als Cholinomimetika, wobei die Verbindungen zum Teil neu sind.

Es wurde gefunden, daß Verbindungen der allgemeinen Formel I

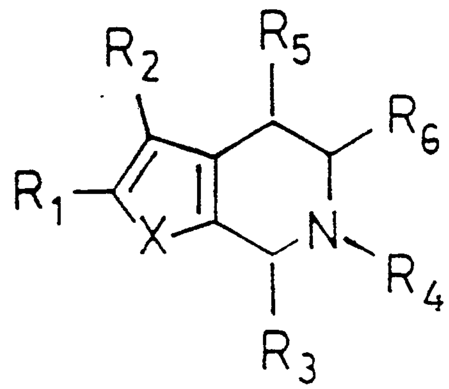

worin

$R_1$ Wasserstoff, verzweigtes oder unverzweigtes Alkyl mit 1 bis 8 Kohlenstoffatomen, worin gegebenenfalls ein $CH_2$ durch C=O ersetzt sein kann, verzweigtes oder unverzweigtes Hydroxyalkyl mit 1 bis 4 Kohlenstoffatomen, verzweigtes oder unverzweigtes Alkoxy mit 1 bis 4 Kohlenstoffatomen, Halogen, Cyano, $COOR'$ worin $R'$ eine verzweigte oder unverzweigte Alkylgruppe mit 1 bis 4 Kohlenstoffatomen bedeutet:

$R_2$ Wasserstoff, Halogen oder verzweigtes oder unverzweigtes Alkyl mit 1 bis 4 Kohlenstoffatomen;

$R_3$ Wasserstoff oder verzweigtes oder unverzweigtes Alkyl mit 1 bis 4 Kohlenstoffatomen;

$R_4$ Wasserstoff oder verzweigtes oder unverzweigtes Alkyl mit 1 bis 4 Kohlenstoffatomen;

$R_5$ Wasserstoff oder verzweigtes oder unverzweigtes Alkyl mit 1 bis 4 Kohlenstoffatomen;

$R_6$ Wasserstoff oder Methyl;

und X Sauerstoff oder Schwefel bedeuten können

sowie gegebenenfalls deren pharmakologisch verträglichen Säureadditionssalze oder ihre quarternären Verbindungen als Arzneimittel mit cholinomimetischen Eigenschaften verwendet werden können.

Im Sinne dieser Erfindung werden als Alkylgruppen -gegebenenfalls auch als Bestandteil anderer funktioneller Gruppen - Methyl, Ethyl, Propyl, Butyl, Pentyl, Hexyl, Heptyl und Octyl wie auch deren Isomere angesehen.

Bevorzugt sind Verbindungen der allgemeinen Formel I, worin $R_1$ eine verzweigte oder unverzweigte Alkylgruppe mit 1 bis 8, besonders bevorzugt 1 bis 4, Kohlenstoffatomen, $R_2$, $R_3$ und/oder $R_5$ Wasserstoff, $R_6$ Wasserstoff und $R_4$ eine verzweigte oder unverzweigte Alkylgruppe mit 1 bis 4 Kohlenstoffatomen bedeuten können.

Bevorzugte Alkylgruppen, auch als Bestandteil anderer Substituenten, sind Methyl, Ethyl, n-Propyl, iso-Propyl, n-Butyl, sec.-Butyl, iso-Butyl und tert.-Butyl, Halogen bedeutet eines der Atome aus der Gruppe Fluor, Chlor, Brom oder Jod.

Besonders bevorzugt sind Verbindungen der allgemeinen Formel I, worin X ein Sauerstoffatom, $R_1$ und $R_4$, die gleich oder verschieden sein können, einen Alkylrest mit 1 bis 4 Kohlenstoffatomen, bevorzugt $R_1$ in der Bedeutung Methyl, Ethyl, n-Propyl oder n-Butyl und $R_4$ Methyl, und $R_2$, $R_3$, $R_5$ und $R_6$ Wasserstoff bedeuten.

Bevorzugte Basen, deren pharmakologisch verträgliche Säureadditionssalze und Quartärverbindungen sind:

2,6-Dimethyl-4,5,6,7,-tetrahydro-furo[2.3-c]pyridin

2,6-Dimethyl-4,5,6,7-tetrahydro-thieno[2,3-c]pyridin

6-Methyl-4,5,6,7,-tetrahydro-thieno[2,3-c]pyridin

6-Methyl-4,5,6,7-tetrahydro-furo[2,3-c]pyridin

2-Methyl-4,5,6,7-tetrahydro-furo[2,3-c]pyridin

2-Ethyl-6-methyl-4,5,6,7-tetrahydro-furo[2,3-c]pyridin

6-Methyl-2-n-propyl-4,5,6,7,-tetrahydro-furo[2.3-c]pyridin
6-Methyl-2-n-butyl-4,5,6,7,-tetrahydro-furo[2.3-c]pyridin
6-Methyl-2-iso-butyl-4,5,6,7,-tetrahydro-furo[2.3-c]pyridin
2-Methyl-4,5,6,7,-tetrahydro-thieno[2,3-c]pyridin
2,3,6-Trimethyl-4,5,6,7,-tetrahydro-thieno[2,3-c]pyridin
6-Ethyl-2-methyl-4,5,6,7,-tetrahydro-thieno[2,3-c]pyridin
2-Chlor-6-methyl-4,5,6,7,-tetrahydro-thieno[2,3-c]pyridin
2-Methyl-6,6-dimethyl-4,5,6,7-tetrahydrothieno[2,3-c]pyridiniumjodid
2-Methyl-6,6-dimethyl-4,5,6,7-tetrahydro-furo[2,3-c]pyridiniumjodid
2-Acetyl-6-methyl-4,5,6,7,-tetrahydro-thieno[2,3-c]pyridin
2-Brom-6-methyl-4,5,6,7,-tetrahydro-thieno[2,3-c]pyridin
3,6-Dimethyl-4,5,6,7-tetrahydro-furo[2,3-c]pyridin
2-Ethyl-6-methyl-4,5,6,7-tetrahydro-furo[2,3-c]pyridin
2-Ethoxycarbonyl-6-methyl-4,5,6,7-tetrahydro-furo[2,3-c]pyridin
2-Acetyl-3,6-dimethyl-4,5,6,7-tetrahydro-furo[2,3-c]pyridin
2-Ethyl-3,6-dimethyl-4,5,6,7-tetrahydro-furo[2,3-c]pyridin
2-Brom-3,6-dimethyl-4,5,6,7-tetrahydro-furo[2,3-c]pyridin
2-Chlor-3,6-dimethyl-4,5,6,7-tetrahydro-furo[2,3-c]pyridin
2-Cyano-6-methyl-4,5,6,7-tetrahydro-furo[2,3-c]pyridin
2-Hyroxymethyl-6-methyl-4,5,6,7-tetrahydro-furo[2,3-c]pyridin

Verbindungen der allgemeinen Formel I sind teilweise bekannt. So offenbar die DE-OS 26 28 045 Tetrahydro-thienopyridine, die ausschließlich in 6- und 7-Stellung substituiert sind als Verbindungen mit antiinflammatorischen Eigenschaften, antiarrhythmischen Eigenschaften wie auch eine Hemmung der Blutplättchenaggregation.

Im Gegensatz zu den Tetrahydro-thienopyridinen war es bislang von den entsprechenden Tetrahydrofuropyridinen der allgemeinen Formel I nicht bekannt, diese als Arzneimittel zu verwenden.

Die Verbindungen der allgemeinen Formel I zeigen muscarine agonistische Eigenschaften und sind damit zur Therapie von Krankheiten mit einer Unterfunktion des cholinergen Systems geeignet.

Die erfindungsgemäßen tertiären Amine der allgemeinen Formel I zeigen Affinitäten zu muscarinen Rezeptoren im ZNS und im Tierexperiment muscarine agonistische Eigenschaften im ZNS.
So zeigt beispielsweise das 2,6-Dimethyl-4,5,6,7-tetrahydro-furo[2,3-c]pyridin Hydrochlorid im EEG (Elektroenzephalogramm) des wachen Kaninchens bei einer Applikation von 3 mg/kg p.o. (1 mg/kg i.v.) eine für Cholinomimetica typische Arousal-Reaktion.

Bei gegen Haloperidol sensibilisierten Affen verstärken die erfindungsgemäßen Verbindungen, so z.B. das 2,6-Dimethyl-4,5,6,7-tetrahydro-thieno[2,3-c]pyridin Hydrochlorid (Dosis $<$ 1 mg/kg i.m) die durch Haloperidol hervorgerufenen Dyskinesien, bzw. lösen zusammen mit unterschwelligen Dosen Dyskinesien aus. Weiterhin zeigen die erfindungsgemäßen Verbindungen bei in vitro Untersuchungen muskarine Bindungseigenschaften, bzw. muskarin agonistische GTP-Shifts (Guanosin-Triphosphat). Birdsall, N.I.M., E.C. Hulme and J.M. Stockton 1984 in T.I.P.S. Supplement, Proc. Internat. Symposium on Subtypes of Muscarinic Receptors, Ed. Hirschowitz, Hammer, Giacchetti, Keirns, Levine; Elsevier p. 4-8.

Die Rezeptorbindungsstudien im muskarinisch cholinergen System wurden an den drei Rezeptorsubtypen gemäß den untenstehenden Literaturzitaten ausgeführt
(Watson, M., H. I. Yamamura and W.R. Roeske 1983, Life Sci. 32, 3001-3011;
Hammer, R., C.P. Berrie, N.I.M. Birdsall, A.S.V. Burgen and E.C. Hulme 1980, Nature 283, 90-92;
Hammer, R., E. Giraldo, G.B. Schiavi, E. Monferini and H. Ladinsky 1986, Life Sci. 38, 1653-1662;).

Die Ergebnisse sind in Tabelle I zusammengefaßt.

Tabelle I

| Ergebnisse der Rezeptorbindungsstudien: | | | | | | | | |
|---|---|---|---|---|---|---|---|---|
| Rezeptorsubtyp | | | | | | $M_1$ | $M_2$ | $M_3$ |
| Organ | | | | | | HIPPOCAMPUS | Herz | Tränendrüse |
| [3H]Ligand | | | | | | Pirenzepin | N-Methylscopolamin | |
| Rezeptorbindung | | | | | | [µMol] | | |
| X | $R_1$ | $R_2$ | $R_3$ | $R_4$ | $R_5$ | | | |
| O | $CH_3$ | H | H | $CH_3$ | H | 8,3 | 6,5 | 24,2 |
| S | $CH_3$ | H | H | $CH_3$ | H | 7,5 | 14 | 53,1 |
| S | H | H | H | $CH_3$ | H | 36,3 | >100 | >100 |
| S | $CH_3$ | H | H | H | H | 39,6 | >100 | >100 |
| S | $CH_3$ | H | H | $CH_3$ | $CH_3$ | 5,8 | 8,4 | 34,1 |
| S | $CH_3$ | H | H | Et | H | 2,2 | 4,5 | 11,4 |
| O | n-Bu | H | H | $CH_3$ | H | 0,5 | 0,7 | % |
| O | Et | H | H | $CH_3$ | H | 1,4 | 1,0 | % |
| O | iso-Bu | H | H | $CH_3$ | H | 0,8 | 2,5 | 2,7 |
| O | $CH_3$ | $CH_3$ | H | Et | H | 3,2 | 3,2 | % |
| $R_6$ = Wasserstoff [Bu = Butyl, Et = Ethyl] | | | | | | | | |

Die erfindungsgemäßen Verbindungen verdrängen den muscarinen Agonisten mit hoher Affinität von der Agonisten Bindungsstelle. Die Durchführung der [3H]cis-Methyldioxolan-Bindungsstudien erfolgen analog zu A Closs et al., Naunyn Schmiedebergs Arch. Pharmacol 335, 372-377 (1987). Die Ergebnisse sind in Tabelle Ia zusammengestellt

Tabelle Ia:

| [3H]Ligand Rezeptorbindung | | | | | | cis-Methyldioxolan Ki[$10^{-9}$ Mol/ltr |
|---|---|---|---|---|---|---|
| X | $R_1$ | $R_2$ | $R_3$ | $R_4$ | $R_5$ | |
| O | n-Bu | H | H | $CH_3$ | H | 39 |
| O | N-Pr | H | H | $CH_3$ | H | 53 |
| O | Et | H | H | $CH_3$ | H | 52 |
| O | $CH_3$ | H | H | $CH_3$ | H | 81 |
| O | n-Pr | H | H | H | H | 160 |
| $R_6$ = Wasserstoff<br>Et = Ethyl, Pr = Propyl | | | | | | |

Aufgrund pharmakologischer Befunde sind die Verbindungen zur Behandlung nachfolgend genannter Krankheiten geeignet: Morbus Alzheimer, senile Demenz, kognitive Störungen und außerdem können die Verbindungen zur Verbesserung der Gedächtnisleistung eingesetzt werden.

Quartäre Verbindungen der allgemeinen Formel I eignen sich besonders zu peripheren Anwendungen, so z.B. zur Glaukombehandlung.

Die Erfindung betrifft ferner neue Verbindungen der allgemeinen Formel I

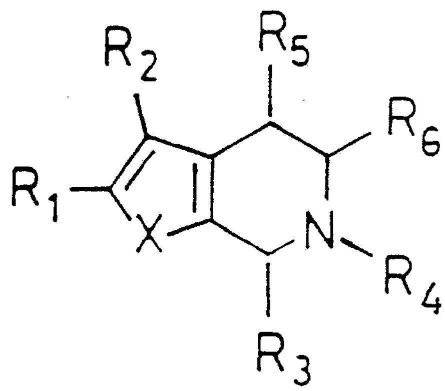

worin
$R_1$ Wasserstoff, verzweigtes oder unverzweigtes Alkyl mit 1 bis 8 Kohlenstoffatomen, worin gegebenenfalls ein $CH_2$ durch $C=O$ ersetzt sein kann, verzweigtes oder unverzweigtes Hydroxyalkyl mit 1 bis 4 Kohlenstoffatomen, eine verzweigte oder unverzweigte Alkoxygruppe mit 1 bis 4 Kohlenstoffatomen, Halogen, Cyano, $COOR'$ worin $R'$ eine verzweigte oder unverzweigte Alkylgruppe mit 1 bis 4 Kohlenstoffatomen bedeutet;
$R_2$ Wasserstoff, Halogen oder verzweigtes oder unverzweigtes Alkyl mit 1 bis 4 Kohlenstoffatomen;
$R_3$ Wasserstoff oder verzweigtes oder unverzweigtes Alkyl mit 1 bis 4 Kohlenstoffatomen;
$R_4$ Wasserstoff oder verzweigtes oder unverzweigtes Alkyl mit 1 bis 4 Kohlenstoffatomen;
$R_5$ Wasserstoff oder verzweigtes oder unverzweigtes Alkyl mit 1 bis 4 Kohlenstoffatomen;
$R_6$ Wasserstoff oder Methyl;
und X Sauerstoff oder Schwefel bedeuten können
sowie gegebenenfalls deren pharmakologisch verträglichen Säureadditionssalze oder deren quarternären Verbindungen mit der Maßgabe, daß im Fall der freien Base wenn
X = Sauerstoff und $R_1$, $R_2$, $R_3$, $R_5$ und $R_6$ Wasserstoff bedeuten - $R_4$ nicht Methyl - ;
X = Schwefel - $R_1$ bis $R_6$ nicht alle Wasserstoff-;
X = Schwefel und $R_1$, $R_2$, $R_3$, $R_5$ und $R_6$ Wasserstoff bedeuten - $R_4$ nicht Methyl sein dürfen.

Bereits P. Mertens et al. J. Org. Chem. 33, 133 (1968) haben vergebliche Syntheseversuche unternommen, die nicht bekannten Tetrahydrofuro[2,3-c]pyridine des Typs

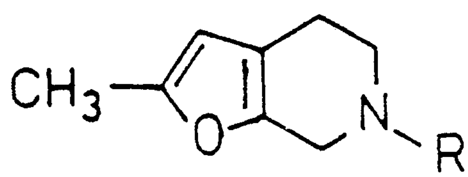

durch eine Ringschlußreaktion geeigneter substituierter Furane, wie z.B. N-(5-Methyl-2-furfuryl)-N-methylglycinethylester darzustellen. Erstmals können nun erfindungsgemäß Tetrahydrofuro[2,3-c]pyridine oben genannter Struktur wie auch ihre Säureadditionssalze erhalten werden.

Die erfindungsgemäßen Verbindungen können nach bekannten Analogieverfahren hergestellt werden.

Ausgehend von 4-Hydroxy substituierten Verbindungen der allgemeinen Formel

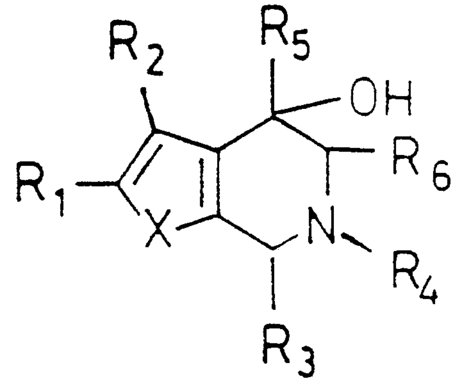

II

worin $R_1$ bis $R_6$ und X die zuvor genannte Bedeutung aufweisen durch Reduktion in Verbindungen der allgemeinen Formel I überführt und anschließend nach bekannten Methoden in ihre Säureadditionssalze oder quartären Salze überführt werden. Hierbei ist es selbstverständlich, daß wenn $R_1$ eine reduzierbare funktionelle Gruppe enthält - wie z.B. eine Hydroxygruppe oder eine Carbonylfunktion (Keton oder Aldehyd) enthält, diese zuvor durch eine Schutzgruppe geschützt werden muß. Die Schutzgruppe wird nach erfolgter Reduktion wieder abgespalten.

Die Reduktion erfolgt zweckmäßigerweise in einem Gemisch aus Eisessig und konzentrierter Salzsäure mit Zinn(II)chlorid bei Temperaturen zwischen Raumtemperatur und dem Siedepunkt des Reaktionsgemisches, bevorzugt bei 50 bis 70° C.

In einer Verfahrensvariante erfolgt die Umsetzung des entsprechenden Alkohols mit einem Gemisch aus Jodwasserstoffsäure und rotem Phosphor in Eisessig bei Siedetemperatur.

Weitere Reduktionsverfahren und Reagenzien sind in einem Übersichtsartikel "Modern methods for the radical deoxygenation of alcohols" von W. Hartwig in Tetrahedron 83; Vol. 39 (16) S. 2609 bis 2645 und in Tetrahedron Letters 82, Vol. 23 (19), S. 2019 beschrieben.

Verbindungen der allgemeinen Formel I, worin $R_5$ Wasserstoff und $R_1$ = einem Acylrest ist, können direkt aus Verbindungen der allgemeinen Formel

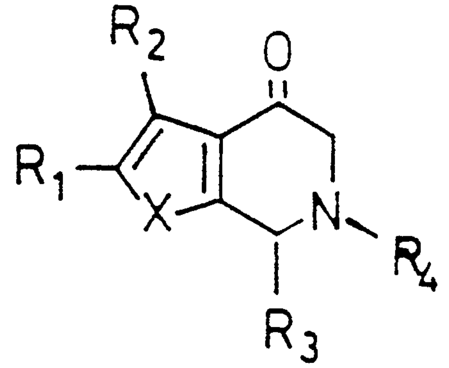

III

erhalten werden, worin $R_1$ bis $R_4$ und X die vorgenannte Bedeutung aufweisen.

Diese Deoxygenierung erfolgt beispielsweise nach der Wolf-Kishner-Methode mit Hydrazinhydrat oder nach deren Huang-Minlon-Variante.

Verbindungen der allgemeinen Formel I mit $R_4$ = Alkyl erhält man durch N-Alkylierung von Verbindungen der allgemeinen Formel I mit $R_4$-Wasserstoff nach bekannten Verfahren, oder Umsetzung mit Carbonylverbindungen unter reduktiven Bedingungen, wie z.B. bei der Leukart-Wallach Reaktion.

Verbindungen der allgemeinen Formel I, in denen $R_1$ und/oder $R_2$ Wasserstoff bedeuten, können durch elektrophile aromatische Substitution in die entsprechenden Alkyl- bzw. Acylderivate überführt werden. Sind $R_1$ und $R_2$ beide Wasserstoff, erfolgt die Substitution vorzugsweise in den 2-Positionen des Moleküls.

Der aromatische Ring ist ebenso nucleophilen aromatischen Substitutionsreaktionen zugänglich. Verbindungen der allgemeinen Formel I( mit $R_1$ = Halogen, wie z.B. Chlor oder Brom, können in die entsprechenden Alkyl- oder Alkoxyverbindungen überführt werden. Die Reaktionsbedingungen der aromatischen Substitution sind bekannt und brauchen nicht näher erläutert werden. Durch Umsetzung mit Kupfer (I) cyanid erhält man

beispielsweise die entsprechenden Nitrile.

N-Allyl-Thiophenderivate der allgemeinen Formel

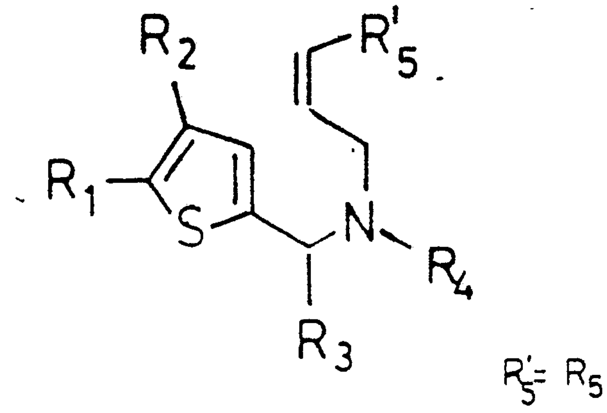

worin $R_1$ bis $R'_5$ unabhängig voneinander Wasserstoff oder Alkyl, wie zuvor definiert, bedeuten, können in Gegenwart von Lewis-Säuren, wie z.B. Aluminiumchlorid, in inerten organischen Lösungsmitteln, wie z.B. halogenierten Kohlenwasserstoffen, z.B. Dichlormethan, zu Verbindungen der allgemeinen Formel I cyclisiert werden.

Die quarternären Verbindungen der allgemeinen Formel I erhält man nach üblichen Verfahren aus den entsprechenden tertiaren Aminen durch Umsetzung mit einem geeigneten Quarternisierungsmittel, wie. z.B. Methyljodid, p-Toluol-sulfonsäuremethylester, in polaren Solventien, wie. z.B. Nitromethan, Acetonitril oder Alkoholen.

Ein weiteres Herstellverfahren für Verbindungen der allgemeinen Formel I ist die N-Alkylierung von Furo- bzw. Thieno[2,3-c]pyridinen der allgemeinen Formel

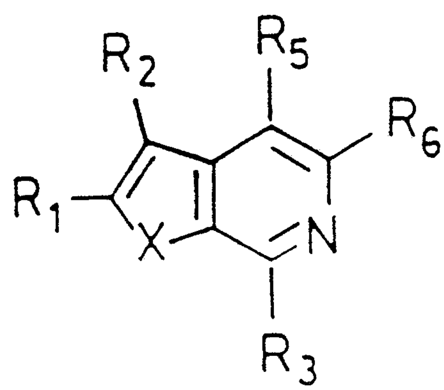

worin X und $R_1$ bis $R_6$ die zuvor genannte Bedeutung aufweisen sowie die daran anschließende Reduktion des Pyridinringes. Bei diesem Verfahren können beispielsweise Reaktionsbedingungen angewendet werden, wie sie von Shiotoni et al, in J. Heterocyclic Chem. 23, 233 (1986) bei der Synthese des 6-Methyl-4,5,6,7-tetrhydro-furo [2,3-c]pyridins angewendet wurden.

Die Alkohole der allgemeinen Formel II sind ebenfalls nach bekannten Verfahren bzw. Analogieverfahren herstellbar, so z.B. durch Reduktion der entsprechenden Ketone mit komplexen Hydriden, wie z.B. Lithiumaluminiumhydrid, in inerten organischen Lösungsmitteln, wie z.B. Diethylether, Tetrahydrofuran u.a..

Ein weiteres Herstellverfahren für die Thienoderivate besteht in der Umsetzung der entsprechenden Formylderivate der allgemeinen Formel, bzw. deren Acetale,

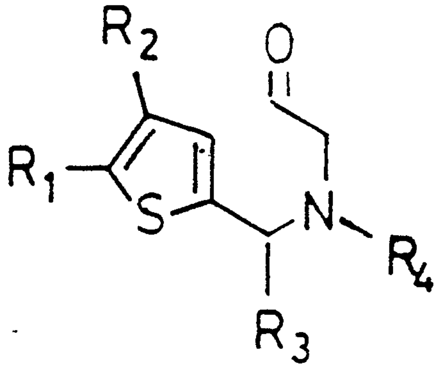

in protischen Reaktionsmedien, wie z.B. in halbkonzentrierter Salzsäure bei Raumtemperatur und anschließender Reduktion zu I.

Die erfindungsgemäßen Tetrahydro-furo- bzw. -thieno[3,2-c]pyridine der allgemeinen Formel I können auf übliche Weise in ihre physiologisch verträglichen Säureadditionssalze überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Bevorzugte Säureadditionssalze sind die Hydrochloride sowie Hydrobromide.

Nach den beschriebenen Verfahren können beispielsweise die Verbindungen der allgemeinen Formel I erhalten werden

Tabelle II

**Erfindungsgemäße Verbindungen der allgemeinen Formel I mit $R_6$ = Wasserstoff**

| Verb. Nr. | X | $R_1$ | $R_2$ | $R_3$ | $R_4$ | $R_5$ | Fp °C der Hydrochloride |
|---|---|---|---|---|---|---|---|
| 1 | O | $CH_3$ | H | H | $CH_3$ | H | 256-258 Zers. |
| 2 | S | $CH_3$ | H | H | $CH_3$ | H | 226 |
| 3 | S | $CH_3$ | H | H | H | H | 201-202 |
| 4 | S | $CH_3$ | H | H | $CH_3$ | $CH_3$ | 200-202 |
| 5 | S | $CH_3$ | H | H | $C_2H_5$ | H | 210-211 |
| 6 | S | Cl | H | H | $CH_3$ | H | 247-248 |
| 7 | O | H | H | H | $CH_3$ | H | 265-266 |
| 8 | O | H | $CH_3$ | H | $CH_3$ | H | |
| 9 | O | H | H | H | $CH_3$ | H | 263 |
| 10 | O | $CH_3$ | H | H | $CH_3$ | H | 256 |
| 11 | O | $C_2H_5$ | H | H | $CH_3$ | H | 215 |
| 12 | O | n-$C_3H_7$ | H | H | $CH_3$ | H | Öl (Base) |
| 13 | O | n-$C_3H_7$ | H | H | H | H | 174-175 |
| 14 | O | n-$C_4H_9$ | H | H | $CH_3$ | H | 116-117 |
| 15 | O | n-$C_4H_9$ | H | H | H | H | 170-172 |
| 16 | O | 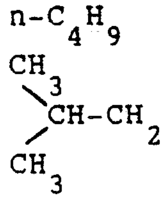 | H | H | H | H | 198-199 |
| 17 | O | " | H | H | $CH_3$ | H | 129-130 |
| 18 | O | H | $CH_3$ | H | $CH_3$ | H | 260-261 |
| 19 | O | $CH_3$ | $CH_3$ | H | $CH_3$ | H | 235-236 |
| 20 | O | $CH_3$ | $CH_3$ | H | $C_2H_5$ | H | 184-185 |

Die Verbindungen der allgemeinen Formel I können allein oder in Kombination mit anderen erfindungsgemäßen Wirkstoffen, gegebenenfalls auch in Kombination mit weiteren pharmakologisch aktiven Wirkstoffen,z.B. weiteren Cerebroaktivatoren zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen,

beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowei ein geschmacksverbesserndes Mittel, z.B. Aromastoffe, wie Vanillin oder Orangenextrakt, enthalten.

Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.
Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Die nachfolgenden Beispiele illustrieren die vorliegende Erfindung ohne sie jedoch in ihrem Umfang zu beschränken:

Pharmazeutische Formulierungsbeispiele

| A) Tabletten | |
|---|---|
| | pro Tablette |
| Wirkstoff | 100 mg |
| Milchzucker | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet , knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) Tabletten | |
|---|---|
| | pro Tablette |
| Wirkstoff | 80 mg |
| Maistärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxy-methylstärke und das Magnesiumstearat, vermischt und verpreßt das Gemisch zu Tabletten geeigneter Größe.

| C) Ampullen | |
|---|---|
| Wirkstoff | 50 mg |
| Natriumchlorid | 10 mg |
| bidestilliertes Wasser q.s. ad | 1,0 ml |

Herstellung:

Der Wirkstoff und das Natriumchlorid werden in bidestilliertem Wasser gelöst und die Lösung in Ampullen steril abgefüllt.

| D) Tropfen | |
|---|---|
| Wirkstoff | 5,0 g |
| p-Hydroxybenzoesäuremethylester | 0,1 g |
| p-Hydroxybenzoesäurepropylester | 0,1 g |
| entmineralisiertes Wasser q.s. ad | 100,0 ml |

Herstellung:

Der Wirkstoff und die Konservierungsmittel werden in demineralisiertem Wasser gelöst und die Lösung filtriert und in Flaschen zu je 100 ml abgefüllt.

Beispiel 1

2,6-Dimethyl-4,5,6,7-tetrahydrofuro[2,3-c]pyridin

10 g (49mmol) 2,6-Dimethyl-4-hydroxy-4,5,6,7-tetrahydrofuro[2,3-c]pyridin (hergestellt nach J. Chem. Soc. Perkin Trans I 1986, Seite 877 ff bzw. DOS 3315157) werden in einer Mischung aus 120 ml Eisessig und 60 ml konz. Salzsäure gelöst und mit 19,1 g (99 mmol) Zinn(II)chlorid versetzt. Dann rührt man 2,5 Stunden bei 65 °C. Anschließend engt man das Reaktionsgemisch ein, nimmt den Rückstand in Wasser

auf, stellt mit konz. Ammoniak unter Eiskühlung alkalisch und extrahiert danach mehrfach mit Essigester. Nach Trocknen und Einengen des Lösungsmittels, wird durch Chromatographie gereinigt (Kieselgel, Fließmittel = Essigester: Methanol: Ammoniak = 10:1:0.5). Die so gereinigte Base wird ins Hydrochlorid überführt und aus Ethanol umkristallisiert.

Ausbeute: 2,6 g (28 % der Titelverbindung als Hydrochlorid vom Fp. 256-258 C (Zers.)

| | | | | | | | | |
|---|---|---|---|---|---|---|---|---|
| Ber. | C | 57,59 | H | 7,51 | N | 7,46 | Cl | 18,89 |
| Gef. | C | 57,31 | H | 7,56 | N | 7,65 | Cl | 18,65 |

Beispiel 2

2,6-Dimethyl-4,5,6,7-tetrahydrothieno[2,3-c]pyridin

2,1

63,5 g (0,45 mol) 2-(N-Methylaminomethyl)-5-methylthiophen werden zusammen mit 47,5 g Natriumcarbonat, 0,5 g Kaliumjodid und 76,3 g (0,5 mol) Chloracetaldehyddiethylacetal in 250 ml wasserfreiem Dimethylformamid 5,5 h bei 130 C gerührt. Diese Suspension filtriert man ab, engt ein und destilliert den Rückstand in Vakuum.

64,7 g (56 %) 5-Methyl-2-(N-methyl-N-2,2-diethoxyethylaminomethyl)thiophen Kp (14 Torr) 150 - 155 C.

2.2

Das in 2.1. hergestellte Amin erhitzt man in 210 ml 6N Salzsäure 3 Stunden unter Rückfluß, kühlt ab, gibt anschließend das Reaktionsgemisch auf Eis, stellt mit Ammoniak alkalisch und extrahiert mehrfach mit Essigester. Nach üblicher Aufarbeitung und Reinigung wird die so enthaltene Base in das Hydrochlorid überführt, das aus Isopropylether umkristallisiert wird.

Man isoliert 22,3 g (49 % d. Th.) des 2,6-Dimethyl-4-hydroxy-4,5,6,7-tetrahydro-thieno[2,3-c]pyridin-hydrochlorids vom Fp. 156 °C.

2.3

Ausgehend von 9,4 g (51 mmol) 2,6-Dimethyl-4-hydroxy-4,5,6,7-tetrahydro-thieno[2,3-c]pyridin-hydrochlorid erhält man analog zu Beispiel 1: 7.3 g (70 %) der Titelverbindung als Hydrochlorid vom Fp. 226 °C

| $C_9H_{13}NS$ x HCl (203.73) | | | | | | | | |
|---|---|---|---|---|---|---|---|---|
| Ber.: | C | 53,06 | H | 6,93 | N | 6,88 | Cl | 17,40 |
| Gef.: | C | 52,85 | H | 6,98 | N | 6,81 | Cl | 17,21 |

Beispiel 3

2,4,6-Trimethyl-4,5,6,7-tetrahydrothieno[2,3-c]pyridin

14,1 g (78 mmol) 5-Methyl-2-(N-allyl-N-methyl-aminomethyl)thiophen werden in 220 ml Dichlorethan gelöst und unter Kühlung mit 22,6 g Aluminiumchlorid versetzt. Danach läßt man das Reaktionsgemisch langsam auf Raumtemperatur erwärmen. Man rührt noch 24 Stunden, gibt das Reaktionsgemisch auf Eis,

stellt alkalisch und trennt die org. Phase ab. Die wässrige Phase wird noch zweimal mit je 200 ml Dichlormethan extrahiert. Nach Trocknen und Einengen der vereinigten organischen Phasen wird an Kieselgel mit Dichlormethan/Methanol (97:3) chromatographiert und die einheitlichen Fraktionen nach Abdampfen des Lösungsmittels mit ethanolischer Salzsäure versetzt und durch Zugabe von Ether gefällt. Die erhaltenen Kristalle werden nochmals aus Ethanol/Ether umkristallisiert.

Man erhält so 0.6 g der Titelverbindung als Hydrochlorid vom Fp. 200 - 202 °C

Beispiel 4

6-Ethyl-2-methyl-4,5,6,7-tetrahydrothieno[2,3-c]pyridin

Das analog Beispiel 2 hergestellte Hydrochlorid des 2-Methyl-4,5,6,7-tetrahydrothieno[2,3-c]pyridins vom Fp. 201-202 °C wird wie folgt alkyliert:

2,5 g (13 mmol) des Hydrochlorids löst man in 100 ml wasserfreiem Dimethyl-formamid gibt 2,8 g (18 mmol) Ethyljodid, 1,2 g Kaliumjodid und 4,2 g Natriumcarbonat zu und rührt 3 Stunden bei 100 °C. Man engt ein, nimmt mit Wasser und Dichlormethan auf, stellt die wässrige Phase alkalisch und arbeitet wie üblich weiter auf. Der dabei erhaltene Rückstand wird an Kieselgel mit Dichlormethan/Methanol (97 : 3) als Eluens chromatographiert. Die isolierte Base wird ins Hydrochlorid überführt, das aus Ethanol umkristallisiert wird.

Ausbeute: 1,2 g (43 %, Fp.: 210 - 211 °C

Beispiel 5

2-Chlor-6-methyl-4,5,6,7-tetrahydrothieno[2,3,-c]pyridin

2,7 g (14 mmol) 6-Methyl-4,5,6,7-tetrahydrothieno[2,3-c]pyridin-hydrochlorid vom Fp. 226 - 228 °C, hergestellt nach Beispiel 2), werden in 90 ml Eisessig gelöst und bei 15 °C werden 2,2 g (16 mmol) Sulfurylchorid zugetropft. Dann rührt man 48 Stunden bei Raumtemperatur, gibt auf Eis, stellt alkalisch und extrahiert mehrfach mit Dichlormethan. Das aus der organischen Phase durch Trocknen und Einengen gewonnene Rohprodukt wird an Kieselgel mit Dichlormethan/Methanol chromatographiert. Die einheitlichen Fraktionen werden eingeengt und ins Hydrochlorid überführt.

Ausbeute 0,5 g; Fp. 247-248 °C (Ethanol)

| $C_8H_{10}ClNS$ x HCl (224,15) | | | | | | | | | |
|---|---|---|---|---|---|---|---|---|---|
| Ber.: | C | 42,87 | H | 4,95 | N | 6,25 | Cl | 31,63 | S | 14,31 |
| Gef.: | C | 42,65 | H | 4,95 | N | 6,07 | Cl | 31,27 | S | 14,38 |

Beispiel 6

2-Methyl-6,6-N,N-dimethyl-4,5,6,7-tetrahydrofuro[2,3-c]pyridiniumjodid

0,5 g (2,7 mmol) 2,6-Dimethyl-4,5,6,7-tetrahydro-furo[2,3-c]pyridin werden in 10 ml Ethanol gelöst und mit 0,72 g Methyljodid versetzt. Nach 3 h Rühren bei Raumtemperatur werden die ausgefallenen Kristalle abgesaugt und dann aus Ethanol umkristallisiert.

Ausbeute 0,52 g Fp. 190 - 193 °C

| Elementaranalyse: $C_{10}H_{16}NOJ$ (293,15) | | | | | | | | |
|---|---|---|---|---|---|---|---|---|
| Ber.: | C | 40,97 | H | 5,50 | N | 4,78 | J | 43,29 |
| Gef.: | C | 41,00 | H | 5,57 | N | 4,95 | J | 43,04 |

Beispiel 7

2-Methyl-6,6-N,N-dimethyl-4,5,6,7-tetrahydrothieno[2,3-c]pyridiniumjodid

In Analogie zu Beispiel 6 werden 2,7 mmol 2,6-Dimethyl-4,5,6,7-tetrahydro-thieno[2,3-c]pyridin gelöst in 10 ml Ethanol mit 0.8 Methyljodid versetzt. Nach erfolgter Aufarbeitung erhält man das entsprechende quartäre Ammoniumsalz als weiße Kristalle vom Schmp. 160 - 161 °C.

Beispiel 8

2-Acetyl-6-methyl-4,5,6,7-tetrahydro-thieno[2,3-c]pyridin-hydrochlorid

3,2 g (21 mmol) 6-Methyl-4,5,6,7-tetrahydro-thieno[2,3-c]pyridin werden in 10 ml Dichlorethan gelöst und mit 3,3 g Aluminiumtrichlorid versetzt. Zur Mischung tropft man 1,7 g Acetylchlorid gelöst in 20 ml Dichlorethan und rührt 16 Stunden. Anschließend schüttet man das Reaktionsgemisch auf Eis, stellt alkalisch und extrahiert die Base mit Dichlormethan und rührt 16 Stunden. Man gibt auf Eis, stellt alkalisch und extrahiert die Base mit Dichlormethan.

Nach üblicher chromatographischer Aufarbeitung, anschließender Überführung in das Hydrochlorid und mehrmaligem Umkristallisieren aus Ethanol werden 0,5 g der Titelverbindung vom Fp. 256 - 257 °C gewonnen.

Beispiel 9

2-(1-Hydroxyethyl)-6-methyl-4,5,6,7-tetrahydro-thieno[2,3-c]pyridin.

4,2 g (0.018 Mol) der in Beispiel 8 beschriebenen Acetylverbindung werden in 100 ml Ethanol und 30 ml Wasser vorgelegt und portionsweise mit 0,4 g (0,011 Mol) Natriumborhydrid versetzt, hierbei läßt man die Temperatur auf 28 °C ansteigen. Es wird anschließend 3 Stunden bei 50 °C gerührt und nochmals 0,4 g Natriumborhydrid zugegeben; nach 2 Stunden wird das Reaktionsgemisch mit 50 ml Eiswasser versetzt und mit Dichlormethan extrahiert. Nach herkömmlicher Aufarbeitung wird der Rückstand der organischen Phase in Ether/Ethanol 2:1 gelöst und unter Zugabe von 2N ethanolischen HCl sauer gestellt. Man erhält 1,7 g (40 % d.Th) der gewünschten Titelverbindungen als Hydrochlorid vom Schmp. 131-132 °C.

Alternativ kann die Reduktion auch in Ether/THF mit Lithiumalanat als Reduktionsmittel durchgeführt werden.

Beispiel 10

2-Brom-6-methyl-4,5,6,7-tetrahydrothieno[2,3-c]pyridin

Zu einer Lösung von 3,0 g (0,016 Mol) 6-Methyl-4,5,6,7-tetrahydro-thieno [2,3-c]-pyridin in 65 ml Wasser wird bei 0 °C eine Lösung aus 4,6 g (0,038 Mol) Kaliumbromid und 2,4 g (0,017 Mol) Brom, gelöst 30 ml Wasser, getropft. Es wird anschließend noch 3 Stunden bei 10 bis 15 °C nachgerührt und die entstandenen Kristalle abfiltriert und mit Eiswasser gewaschen. Diese werden dann in einer verdünnten Ammoniumhydroxidlösung aufgenommen und mehrmals mit Dichlormethan extrahiert. Nach herkömmlicher

Aufarbeitung wird der Rückstand der organischen Phase in 20 ml Methanol gelöst und mit 2N ethanolischen HCL sauer gestellt. Man erhält 1,7 g (40 % d.Th) der Titelverbindung als Hydrochlorid in Form farbloser Kristalle vom Schmp. 260-261 °C.

Beispiel 11

3-Brom-2,6-dimethyl-4,5,6,7-tetrahydrothieno[2,3-c]pyridin

In Analogie zu Beispiel 10 erhält man ausgehend von 2,6-Dimethyl-4,5,6,7-tetrahydro-thieno[2,3-c]-pyridin durch Bromierung die Titelverbindung als hellgelbes kristallines Hydrochlorid in 49 %ige Ausbeute vom Schmelzpunkt 300 bis 302 °C.

Beispiel 12

3,6-Dimethyl-4,5,6,7-tetrahydrofuro[2,3-c]pyridin

Ausgangsverbindung:

3,6-Dimethylfuro[2,3-c]pyridiniumjodid.

2,2 g (16,5 mmol) 3-Methylfuro[2,3-c]pyridin werden mit 11,7 g (82,5 mmol) Methyljodid in 10 ml wasserfreiem Acetonitril umgesetzt (H. Morita et al., J. Heterocyclic Chem. 23, 549 (1986)).
Man erhält nach üblicher Aufarbeitung 4 g (88,9 % d.Th.) grau-gelbe Kristalle vom Schmp. 177-178 °C.

Endverbindung:

2 g (7,2 mmol) des zuvor hergestellten Jodids werden in 50 ml wasserfreiem Methanol vorgelegt, anschließend werden bei Raumtemperatur insgesamt 0,75 g Natriumborhydrid portionsweise zugegeben. Nach der hydrolytischen Aufarbeitung isoliert man aus der organischen Phase ($CH_2Cl_2$) durch Zugabe von HCl in Ether 0,9 g (66,3 % d.Th) der Titelverbindung als Hydrochlorid vom Schmelzpunkt 287 - 288 °C.

Beispiel 13

2,5,6-Trimethyl-4,5,6,7-tetrahydro-thieno[2,3-c]pyridin

10,2 g (50 mmol) 2,5,6-Trimethyl-4-hydroxy-4,5,6,7-tetrahydrothieno[2,3-c]pyridin werden mit 18,9 g (100 mmol) wasserfreiem Zinn-II-chlorid in 60 ml wasserfreiem Eisessig umgesetzt. Nach sechs Stunden wird hydrolytisch ($NH_4OH$) aufgearbeitet. Aus der organischen Phase ($CH_2Cl_2$) erhält man nach der Umkristallisation aus Essigester/Ether (8 : 2) 5,6 (51 % d.TH.) der Titelverbindung vom Schmelzpunkt 165-166 °C.

## Ansprüche

1. Tetrahydropyridine der allgemeinen Formel I

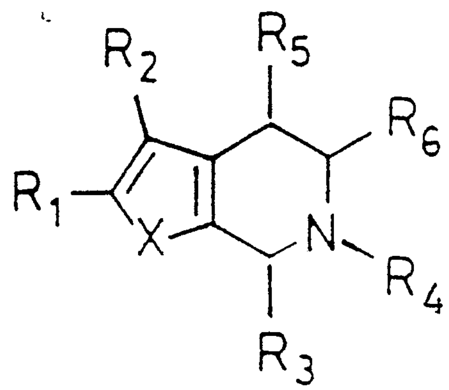

worin
$R_1$ Wasserstoff, verzweigtes oder unverzweigtes Alkyl mit 1 bis 8 Kohlenstoffatomen, worin gegebenenfalls ein $CH_2$ durch C=O ersetzt sein kann, verzweigtes oder unverzweigtes Hydroxyalkyl mit 1 bis 4 Kohlenstoffatomen, eine verzweigte oder unverzweigte Alkoxygruppe mit 1 bis 4 Kohlenstoffatomen, Halogen, Cyano, COOR′ worin R′ eine verzweigte oder unverzweigte Alkylgruppe mit 1 bis 4 Kohlenstoffatomen bedeutet;
$R_2$ Wasserstoff, Halogen oder verzweigtes oder unverzweigtes Alkyl mit 1 bis 4 Kohlenstoffatomen;
$R_3$ Wasserstoff oder verzweigtes oder unverzweigtes Alkyl mit 1 bis 4 Kohlenstoffatomen;
$R_4$ Wasserstoff oder verzweigtes oder unverzweigtes Alkyl mit 1 bis 4 Kohlenstoffatomen;
$R_5$ Wasserstoff oder verzweigtes oder unverzweigtes Alkyl mit 1 bis 4 Kohlenstoffatomen;
$R_6$ Wasserstoff oder Methyl;
und X Sauerstoff oder Schwefel bedeuten können
sowie gegebenenfalls deren pharmakologisch verträglichen Säureadditionssalze oder deren quarternären Verbindungen mit der Maßgabe, daß im Fall der freien Base wenn
X = Sauerstoff und $R_1$, $R_2$, $R_3$, $R_5$ und $R_6$ Wasserstoff bedeuten - $R_4$ nicht Methyl - ;
X = Schwefel und $R_1$ bis $R_6$ nicht alle Wasserstoff -;
X = Schwefel und $R_1$, $R_2$, $R_3$, $R_5$ und $R_6$ Wasserstoff bedeuten - $R_4$ nicht Methyl sein können.

2. Tetrahydropyridine der allgemeinen Formel I gemäß Anspruch I, dadurch gekennzeichnet, daß $R_2$, $R_3$, $R_5$ und $R_6$ Wasserstoff, $R_1$ eine verzweigte oder unverzweigte Alkylgruppe mit 1 bis 4 Kohlenstoffatomen, $R_4$ Wasserstoff oder Methyl und X Sauerstoff oder Schwefel bedeuten können sowie gegebenenfalls deren pharmakologisch verträglichen Säureadditionssalze wie auch deren quarternären Verbindungen

3. Verfahren zur Herstellung von Verbindungen der allgemeinen Formel I

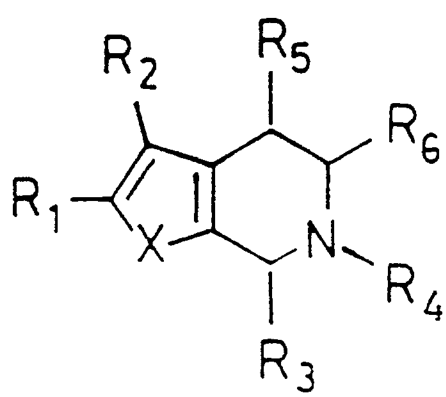

worin
$R_1$ Wasserstoff, verzweigtes oder unverzweigtes Alkyl mit 1 bis 8 Kohlenstoffatomen, worin gegebenenfalls ein $CH_2$ durch C=O ersetzt sein kann, verzweigtes oder unverzweigtes Hydroxyalkyl mit 1 bis 4

Kohlenstoffatomen, verzweigtes oder unverzweigtes Alkoxy mit 1 bis 4 Kohlenstoff- atomen, Halogen, Cyano, COOR′ worin R′ eine verzweigte oder unverzweigte Alkylgruppe mit 1 bis 4 Kohlenstoffatomen bedeutet;

$R_2$ Wasserstoff, Halogen oder verzweigtes oder unverzweigtes Alkyl mit 1 bis 4 Kohlenstoffatomen;

$R_3$ Wasserstoff oder verzweigtes oder unverzweigtes Alkyl mit 1 bis 4 Kohlenstoffatomen;

$R_4$ Wasserstoff oder verzweigtes oder unverzweigtes Alkyl mit 1 bis 4 Kohlenstoffatomen;

$R_5$ Wasserstoff oder verzweigtes oder unverzweigtes Alkyl mit 1 bis 4 Kohlenstoffatomen;

$R_6$ Wasserstoff oder Methyl; und X Sauerstoff oder Schwefel bedeuten können sowie gegebenenfalls deren pharmakologisch verträglichen Säureadditonssalze oder ihre quaternären Verbindungen dadurch gekennzeichnet, daß man eine Verbindung der allgemeinen Formel II

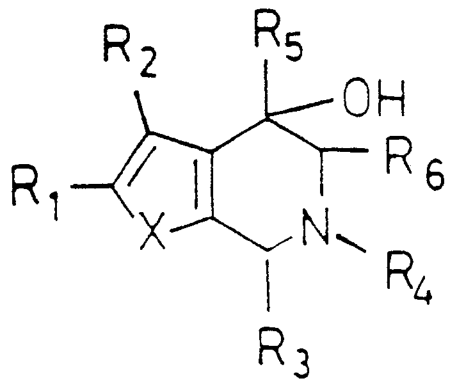

II

worin X und $R_1$ bis $R_6$ die zuvor genannte Bedeutung aufweisen, wobei eventuelle Carbonyl-und Hydroxylreste durch Schutzgruppen geschützt sind, reduziert, gegebenenfalls die Schutzgruppen abspaltet, anschließend gegebenenfalls in ihre pharmokologisch unbedenklichen Säureadditionssalze oder in ihre quarternären Salze überführt.

4. Verwendung von Verbindungen der allgemeinen Formel

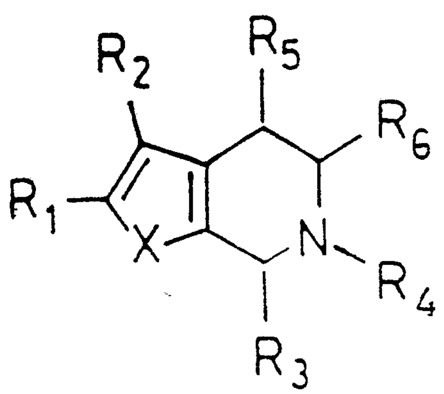

worin

$R_1$ Wasserstoff, verzweigtes oder unverzweigtes Alkyl mit 1 bis 8 Kohlenstoffatomen, worin gegebenenfalls ein $CH_2$ durch C=O ersetzt sein kann, verzweigtes oder unverzweigtes Hydroxyalkyl mit 1 bis 4 Kohlenstoffatomen, verzweigtes oder unverzweigtes Alkoxy mit 1 bis 4 Kohlenstoff- atomen, Halogen, Cyano, COOR′ worin R′ eine verzweigte oder unverzweigte Alkylgruppe mit 1 bis 4 Kohlenstoffatomen bedeutet;

$R_2$ Wasserstoff, Halogen oder verzweigtes oder unverzweigtes Alkyl mit 1 bis 4 Kohlenstoffatomen;

$R_3$ Wasserstoff oder verzweigtes oder unverzweigtes Alkyl mit 1 bis 4 Kohlenstoffatomen;

$R_4$ Wasserstoff oder verzweigtes oder unverzweigtes Alkyl mit 1 bis 4 Kohlenstoffatomen;

$R_5$ Wasserstoff oder verzweigtes oder unverzweigtes Alkyl mit 1 bis 4 Kohlenstoffatomen;

$R_6$ Wasserstoff oder Methyl;

und X Sauerstoff oder Schwefel bedeuten können

sowie gegebenenfalls deren pharmakologisch verträglichen Säureadditionssalze oder ihre quarternären

Verbindungen zur Herstellung eines Arzneimittels mit cholinomimetischen Eigenschaften.

5. Verwendung von 2,6-Dimethyl-4,5,6,7-tetrahydro-furo[2,3-c] pyridin sowie seiner pharmakologisch verträglichen Säureadditionssalze oder seiner quartären Salze als Arzneimittel mit cholinonomimetischen Eigenschaften.

6. Pharmazeutische Zubereitung enthaltend eine Verbindung der allgemeinen Formel I, gemäß Anspruch 1 oder 4 sowie übliche pharmakologisch verträgliche Hilfs- und Trägerstoffe.

7. Verwendung einer Verbindung der allgemeinen Formel I, gemäß Anspruch 1 oder 4 zur Herstellung eines Arzneimittels mit cholinomimetischer Wirkung.

8. Verfahren zur Herstellung von Verbindungen der allgemeinen Formel I gemäß Anspruch 1 oder 2, dadurch gekennzeichnet, daß man ein Pyridinderivat der allgemeinen Formel worin $R_1$ bis $R_6$ die zuvor genannte Bedeutung aufweise mit einem Alkylierungsreagenz der allgemeinen Formel

$R_4$ - Y

worin $R_4$ eine verzweigte oder unverzweigte Alkylgruppe mit 1 - 4 Kohlenstoffatomen und Y eine Austrittsgruppe bedeutet umsetzt und anschließend die erhaltene N-Alkylverbindung reduziert.

9. Verfahren zur Herstellung von Verbindungen der allgemeinen Formel I, gemäß Anspruch 3 mit $R_5$ = Wasserstoff und X = Schwefel, dadurch gekennzeichnet, daß man eine Verbindung der allgemeinen Formel

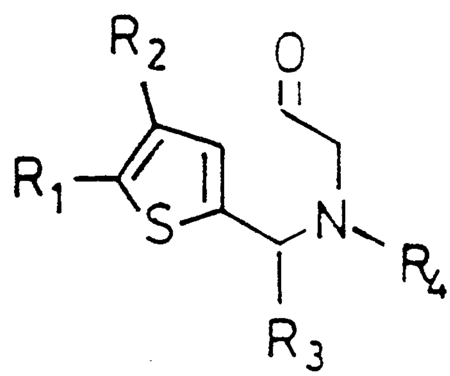

worin $R_1$ bis $R_4$ wie zuvor definiert sind, in protischen Reaktionsmedien ringschließt und anschließend reduziert.

10. Verfahren zur Herstellung von Verbindungen der allgemeinen Formel I gemäß Anspruch 3 mit X = Schwefel, dadurch gekennzeichnet, daß man eine Verbindung der allgemeinen Formel

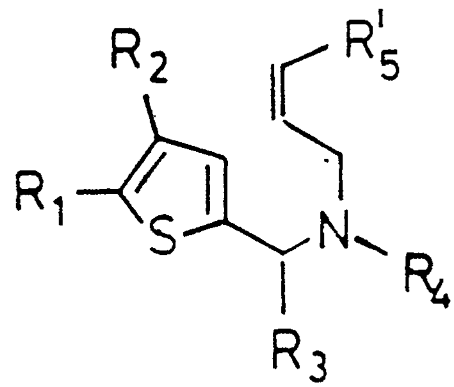

in Gegenwart von Lewis-Säuren cyclisiert.